(19) Europäisches Patentamt
European Patent Office
Office européen des brevets

(11) **EP 2 497 424 A1**

(12) **EUROPEAN PATENT APPLICATION**

(43) Date of publication:
**12.09.2012 Bulletin 2012/37**

(51) Int Cl.:
*A61B 6/02* (2006.01)  *G03B 42/02* (2006.01)

(21) Application number: **11157111.3**

(22) Date of filing: **07.03.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Agfa Healthcare**
**2640 Mortsel (BE)**

(72) Inventor: **Behiels, Gert**
**2640 Mortsel (BE)**

(54) **Radiographic imaging method and apparatus.**

(57)    Method and apparatus for generating an x-ray image of an elongate body in direct radiography by generating a plurality of partial x-ray images of said elongated body and by stitching these partial images.

**FIG. 1**

EP 2 497 424 A1

Printed by Jouve, 75001 PARIS (FR)

**Description**

FIELD OF THE INVENTION

The present invention relates to a method and apparatus for generating an x-ray image of an elongate body in direct radiography by generating a plurality of partial x-ray images of said elongated body and by stitching these partial images.

BACKGROUND OF THE INVENTION

In X-ray radiography an x-ray image of an elongate body, such as the entire spine or the legs of a patient, may have to be obtained.

In Computed Radiography (CR), such a long length image is generated by subjecting a number of Imaging Plates (IP), such as photo-stimulable phosphor plates, which are organized in a partially overlapping disposition to an x-ray image of the elongate body. Each of the imaging plates carries an image of a part of the elongate body. After exposure, the individual imaging plates are read out so as to obtain partial images of the elongate body and finally a long length image is created by stitching these partial images. Accurate alignment and measurement can be obtained by superimposing a grid of radiation attenuating material covering the region to be imaged and correcting and aligning the partial images to reconstruct the geometry of said grid. Such methods are described in European patent applications EP0919856 and EP0866342.

In recent years, Digital Radiography (DR) has become a valuable alternative for CR. The flat panel detectors (FPD) used in DR are more costly than the IP's for CR, so an alternative to the one-shot long length imaging technique of CR using multiple Imaging Plates is needed. This is achieved by taking plural partial images of an elongate body by moving the position of the FPD while tilting the X-ray tube or moving the X-ray tube parallel to the FPD.

It is an aspect of the present invention to create an image of the total elongate body from the partial images in an accurate way permitting length and angular measurements on the composed image.

SUMMARY OF THE INVENTION

The above-mentioned aspects are realized by a method and apparatus having the specific features set out in the independent claims.

Specific features for preferred embodiments of the invention are set out in the dependent claims.

Further advantages and embodiments of the present invention will become apparent from the following description and drawings.

Long length images are mostly taken to perform length and angle measurements on the subject across an area larger than a single FPD. It is therefore important to create an image where the alignment of the partial images of the subject and the calibration is accurate.

In long length imaging, the elongate image is formed by stitching partial images of the elongate body which are taken at plural positions by moving the position of the FPD. In order to support the subject being imaged, a barrier may be placed between the subject and the FPD. This barrier has the purpose to stabilize the subject to minimize movement, to protect the subject from contact with the moving FPD. It can also be used to attach an object with known geometry which in accordance with the present invention is used to align the partial images. Attached to this barrier, multiple rulers can be applied to determine the region to be imaged and the distance of the subject to the barrier.

According to the method of the present invention the radiation image of an object of known geometry is detected and the information on the geometry of this object in the detected radiation image is used to geometrically correct the individual partial images before stitching. The stitch method may again use the geometry of the detected image of the object of known geometry in each of the partial image to stitch the images to form one large image.

The method of the present invention thus comprises the steps of

- generating partial radiation images by multiple shot irradiation and read out of a direct radiography detector, each of the partial radiation images comprising part of the radiation image of the elongate body and part of the radiation image of an object of known geometry superimposed on the radiation image of the elongate body,

- calculating parameters of a geometric transformation expressing the relation between detected positions of locations of the object in a partial radiation image and expected positions of the locations in a partial radiation image,

- projecting the partial images onto a reference plane by applying the geometric transform to its pixels so as to obtain warped partial images,

- stitching the warped partial images so that the image of the object of known geometric dimensions is reconstructed.

In one embodiment the object of known geometry is a grid consisting of X-ray attenuating wires which intersect at a given interval, preferably the interval is 5 by 5 cm.

Alternatively the object of known geometry may consist of a grid consisting of X-ray attenuating crosses positioned at a given interval, preferably an interval of 5 by 5 cm.

In a specific embodiment two types of crosses are provided. All crosses are preferably positioned at a first interval of preferably 5 x 5 cm and the crosses of a second type are positioned at a second interval of preferably 10 x 10 cm.

The geometric transformation used in the present invention is preferably implemented using thin plate spline.

However, alternative implementations are possible, such as piece-wise linear separable de-skewing.

The invention further discloses a X-ray radiographic apparatus for image creation of an elongate body which comprises:

- an X-ray imaging unit including an X-ray flat panel detector,
- means for moving said flat panel detector;
- an X-ray generation unit including an X-ray source,
- means for moving said X-ray source,
- an imaging area setting device capable of setting an imaging area for imaging an elongate body;
- a position determination device for determining a plurality of positions for the X-ray generation unit and the X-ray imaging unit, said positions delineating a plurality of partial imaging areas in said imaging area which overlap with a configured amount,
- an object of known geometry provided between said X-ray imaging unit and X-ray generation unit, said object comprising parts of X-ray attenuating material distinguishable in images generated by said X-ray imaging unit;
- at least one control device for controlling said X-ray generation unit and said X-ray imaging unit so that both units are moved sequentially to said positions delineating partial image areas and that partial radiation images of part of said body and part of said object are generated in each of said positions;
- a processing device for generating an elongate image from the generated partial images.

A movable diaphragm may be provided to adjust the field of view of the x-ray source.

The position determining device is capable of generating the positions and, when applicable, also the diaphragm settings of the x-ray generation unit and the x-ray imaging unit such that the field of view area for imaging (the part of the elongate body to be imaged) is captured by a plurality of images acquired by the flat panel detector.

The processing unit combines the acquired partial images to generate a long length image which is an image of the complete field of view area. For this purpose the processing unit calculates parameters of a geometric transformation expressing the relation between detected positions of locations of the object in a partial radiation image and expected positions of these locations in a partial radiation image. Next the partial images are projected onto a reference plane by applying the geometric transform to its pixels so as to obtain warped partial images. The warped partial images are then stitched so that the image of said object of known geometric dimensions is reconstructed

All units can be combined in a single device or implemented in different devices. In other words, given a desired field of view area as input, the computing unit computes the positions and the diaphragm of the X-ray source and the positions of the flat panel detector. The controlling unit positions the X-ray source and flat panel detector and captures the images at each of the positions. The result is a set of images, each capturing a part of the complete field of view area and as a union covering the complete field of view area.

The method and apparatus of the present invention make use of the radiation image of an object of known geometry present in a partial image to transform the partial image to a representation which is similar to an image taken with a flat panel detector defined in a plane relative to the object of known geometry.

The object of known geometry at least partially covers each of the field of view areas of each exposure of part of the elongate body. Based on the known geometry, the processing unit may detect elements of the object of known geometry and aligns the partial or transformed images in such a way that the part of the object of known geometry is correctly represented in the long length image.

BRIEF DESCRIPTION OF THE DRAWINGS

Figure 1 illustrates the parallax effect in the gray area and the different projection order for the circles if the radiation source changes position,

Figure 2 illustrates a radiographic image acquisition device wherein an X-ray generation unit is suspended on the ceiling supporting a vertical movement of the X-ray generation and the X-ray imaging unit and supporting a rotation of the X-ray generation unit,

Figure 3 illustrates a radiographic image acquisition apparatus where the X-ray generation unit is mounted on the floor supporting a vertical movement of the X-ray generation and the X-ray imaging unit and supporting a rotation of the X-ray generation unit,

Figure 4 illustrates a radiographic image acquisition apparatus where the X-ray generation unit and X-ray imaging unit is mounted on the floor in a single assembly supporting a vertical movement and rotation of this assembly,

Figure 5 illustrates a radiographic image acquisition apparatus where the X-ray generation unit and X-ray imaging unit is mounted on the floor in a single assembly supporting a vertical movement and rotation of this assembly in combination with a rotation of the X-ray imaging unit,

Figure 6 is a representation of a geometric transformation when the middle point in a fixed grid is moved somewhat lower and to the right,

Figure 7 is a representation of the geometric transformation and the result of a deformed rectangular grid warped back to the original positions,

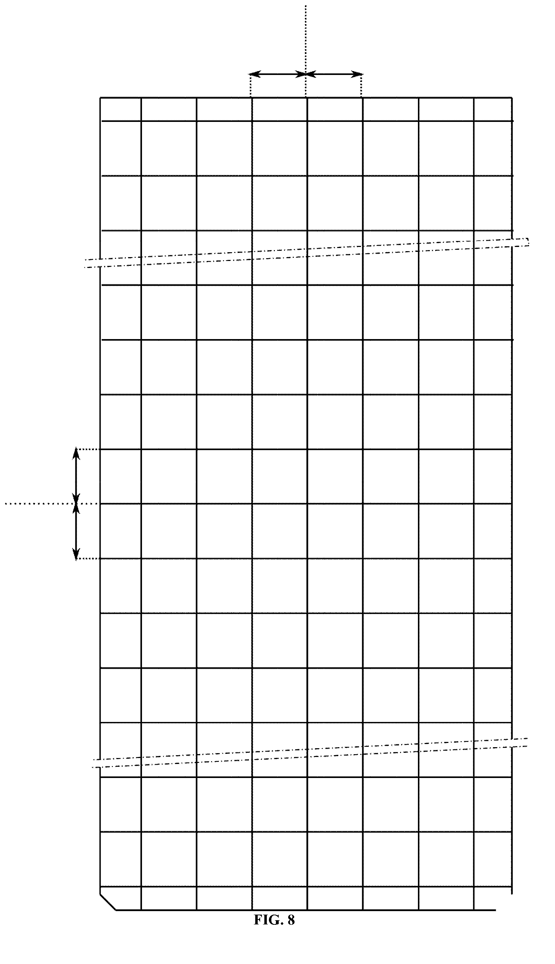

Figure 8 is a schematic drawing of a grid consisting of X-ray attenuating wires which intersect at an interval of 5 by 5 cm which can serve as an object of known geometry,

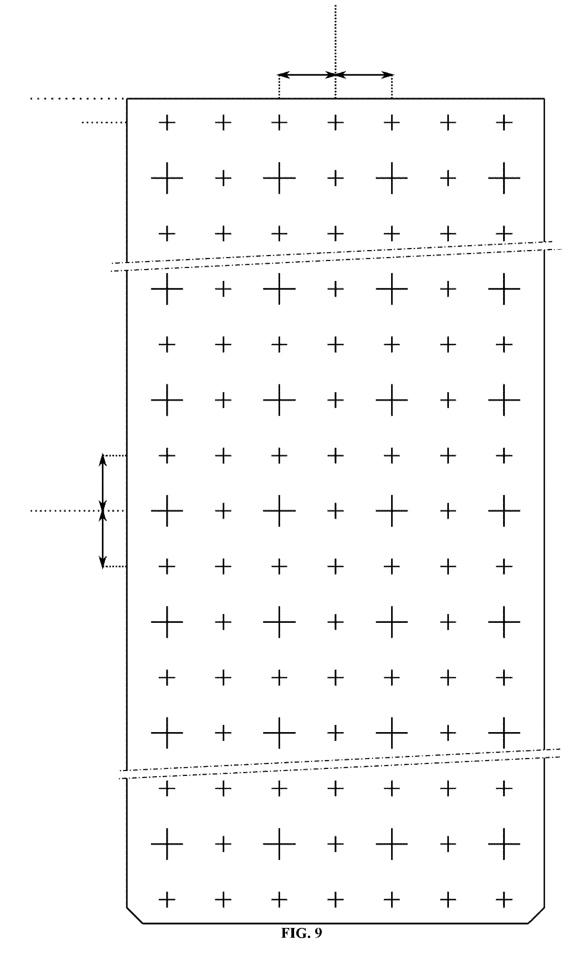

Figure 9 is a schematic drawing of a grid consisting of X-ray attenuating crosses positioned at an interval of 5 by 5 cm wherein the crosses positioned at an interval of 10 by 10 cm are different in size than the non overlapping crosses positioned at an interval of 5 by 5 cm.

DETAILED DESCRIPTION OF THE INVENTION

Acquiring partial images

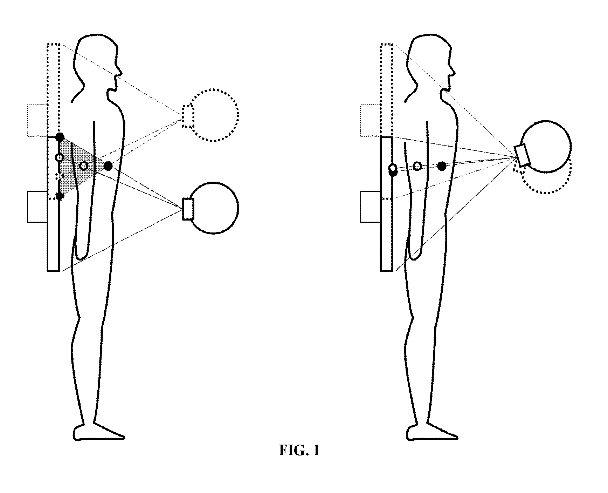

The method of the present invention is aimed to generate a long length image suitable for length and angle measurements on the imaged subject across an area larger than a single flat-panel-detector. The measurements are preferably accurate in all planes, not subjective to errors introduced by the parallax effect. Due to the so-called parallax effect points residing in a different plane are projected differently if the illumination (radiation) source is positioned differently. Not only are objects being projected on different positions in a single reference plane, the projection order may also change for objects in different planes (see the gray area with circles on the left drawing in figure 1). To eliminate this effect, it is advisable to position the illumination source on the same location when taking different partial images. However, even when the illumination source position is not fixed, the method of this invention can still be applied.

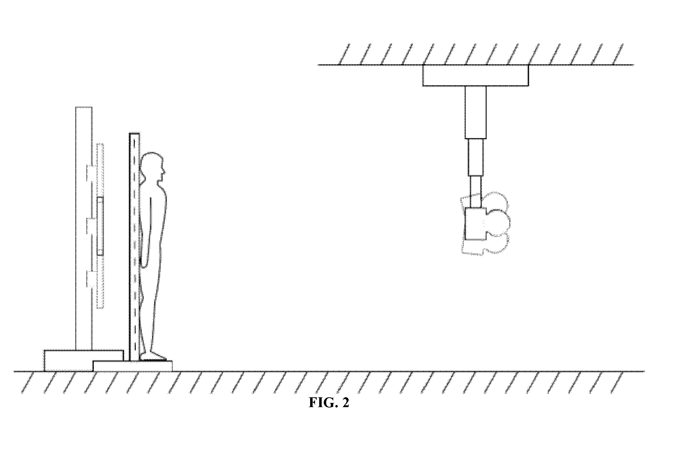

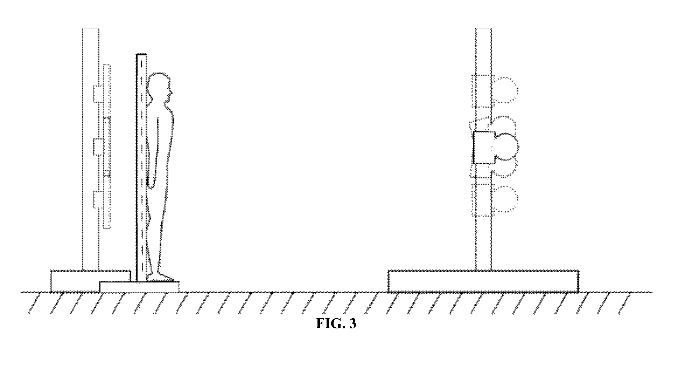

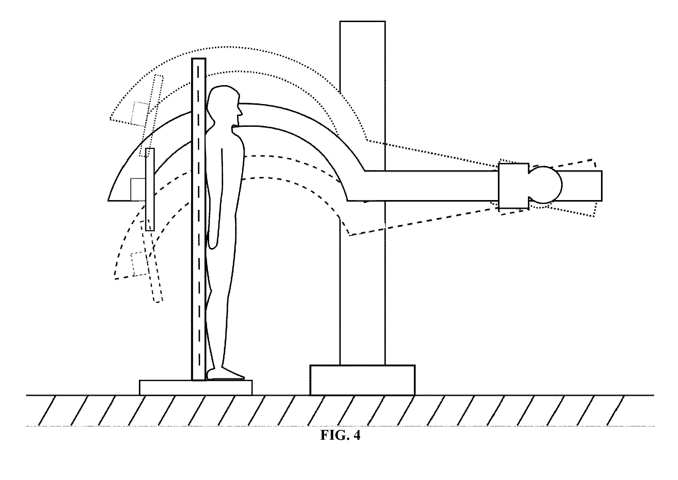

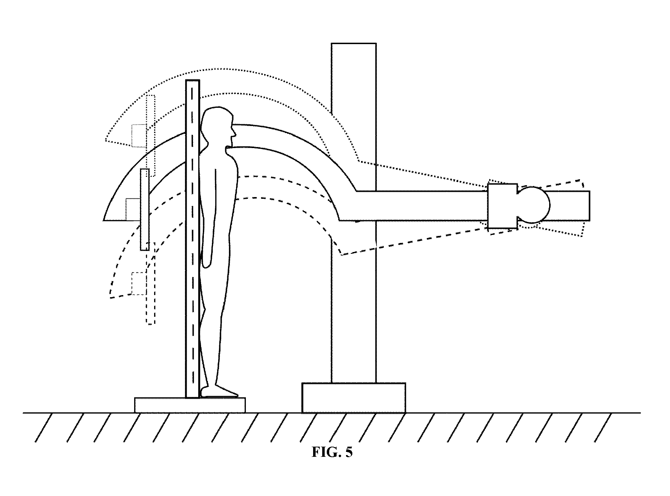

To generate an image covering an area bigger than a single flat panel detector for DR, the following options exist: use more than one flat panel detector and stack them similar to the method applied in computed radiography and described e.g. in EP0919856 or use a special geometry setup for DR such as described in DE102007025448, or use a single flat panel detector and move it to the different positions so as to record a multiplicity of partial images together covering the area of the elongate body. Since the cost of a single flat panel detector with large dimensions covering the total length of an elongate body currently is too high, the method of moving the flat panel detector so as to record a number of partial images of the elongate body is preferably chosen. Several applicable set-ups for generating such partial images are shown in figures 2 to 5.

This choice has two main consequences: multiple exposures are taken during a certain time interval and the patient must stand away from the detector to avoid collision. During the time of the exposures, the patient ideally should not move.

To protect the patient from a collision with the moving imaging unit, a patient barrier may be placed between the patient and the imaging unit. If designed properly, the patient barrier described higher can support the patient to prevent the patient from moving.

When using such a setup, it is clear that all images will be magnified because of the distance between the patient and the detector. If this distance is known, this magnification factor is computed as

$$ERMF = \frac{SID}{SOD}$$

where ERMF stands for Estimated Radiographic Magnification Factor, SID for Source-to-Image Distance and SOD for Source-to-Object Distance where the object represents the patient. The distance between detector and patient (OID) is given by

$$OID = SID - SOD \ .$$

Often the SOD is not known e.g. because of variations of patient thickness and variations in the placement of the patient barrier with respect to the detector.

If an object of known geometry is captured in each of the exposures generating partial images, the magnification factor can be estimated from the image content for each of the partial images independently. Furthermore, if we know how the object of known geometry is projected on a reference plane close to or in the plane in which the patient is positioned, we can compensate for all perspective and other distortions caused by inaccurate alignment or positioning of the flat panel detector.

Therefore in one embodiment of the present invention the object of known geometry is in the form of a grid of x-ray attenuating material which can be used to calibrate the individual images and transform them to a reference plane, in one embodiment being the plane of the grid itself.

To minimize the differences between measurements performed in the grid's reference plane and the measurements of the actual imaged patient object, this grid should preferably be placed as close as possible to the patient. To achieve this, the grid is preferably designed as the object in the patient barrier which supports the patient. In normal imaging conditions, the patient leans against the plate containing the grid, as such the distance between patient and grid is minimal. The design of the grid also allows correct image stitching as explained below.

Since the partial images are acquired within a certain time interval, it is preferable to optimize and automate the acquisition of the partial images. A controlling unit can be used to co-ordinate the positioning of the X-ray generation unit and the X-ray imaging unit, the preparation of the X-ray imaging unit, the activation of the X-ray generation and read-out of the X-ray imaging unit. The optimization is preferably tuned to minimize the complete time for the acquisition of all partial images. All other processing related operations can be postponed to a stage where all images are already acquired.

A specific embodiment of the image acquisition steps of the method of the present invention are summarized as follows: first the X-ray generation unit and X-ray imaging unit are positioned to a default position which allows the placement of the patient barrier. Secondly, the patient barrier containing a calibration and stitching grid is placed to a position close to the detector and the patient is placed against this patient barrier. Thirdly, after input of the desired area to be imaged, the partial images are acquired (one after the other, as fast as possible to prevent patient movement) and sent to a device which is capable of calibrating and stitching the partial images to generate an elongate (or complete) image. Optionally, this device also allows the generated elongate image to be displayed or corrected before sending it to an archive or diagnostic workstation.

Transforming the image

This module will transform the partial images read out of the detector such that they are projected onto a reference plane which is defined in relation to the object of known geometry. By doing so, the differences in magnification factors and perspective deformations between the partial images can be compensated. After such compensation, the resulting warped images can be stitched together as if they were recorded with the X-ray source positioned at the same location for the different partial images. In the proposed setup where a grid is used, the reference plane is preferably the plane of the grid itself.

There are many ways to obtain such a transformation, thin plate splines being one them. It is sufficient to detect reference locations in the image of the object of known geometry and map these reference locations to their corresponding location in the above-mentioned reference plane. The resulting thin-plate-spline transform consists of the affine transformation and coefficients which model the non-rigid deformation.

Next, to construct the image in the reference plane, the position of each pixel of this image is mapped to the original image using the thin-plate-spline and the pixel value at the mapped position in the original image is extracted. Because this mapped position will not always correspond with the position of a pixel value, an interpolation technique can be used to estimate the intensity value.

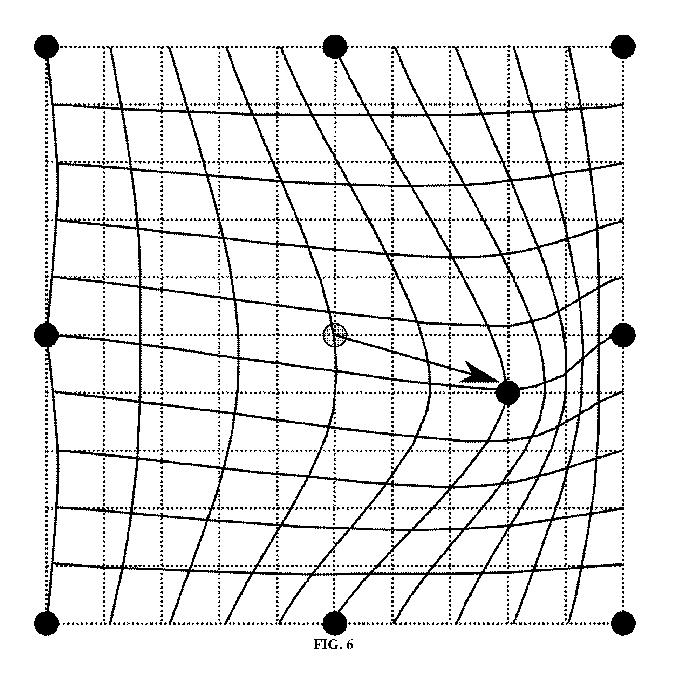

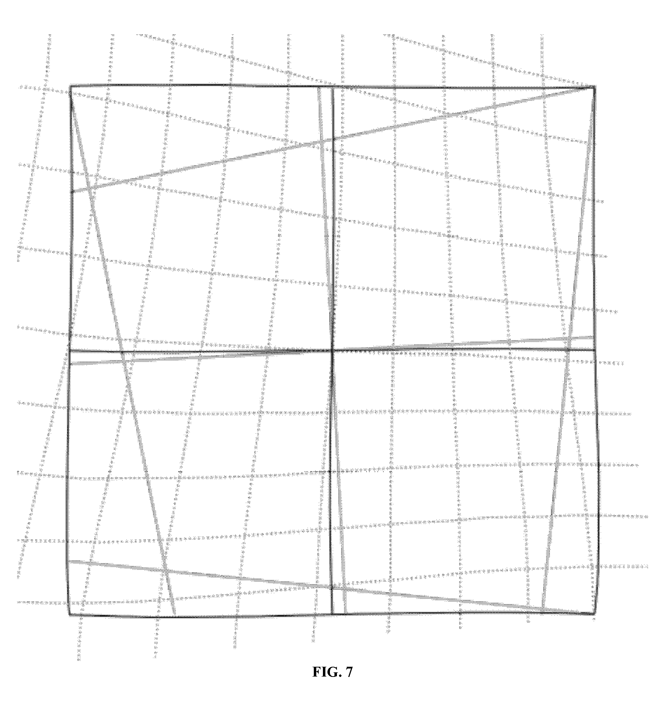

In figure 6, a geometric transformation is represented for a grid of 3x3 points where the middle point is moved somewhat lower to the right. A more realistic configuration is found in figure 7. Here the acquired image is represented by the solid gray lines. Under the assumption that the lines are a representation of a rectangular grid, the intersections are mapped to their corresponding coordinates on the grid. The geometric transformation is illustrated as the dotted lines which maps the gray lines on the solid black lines in the figure resulting in an almost perfect rectangular reconstruction of the grid. It is obvious that more specific deformation models can be used to estimate the deformation of the grid (e.g. piece-wise linear separable de-skewing as described in EP0919856).

If the object of known geometry is a grid consisting of X-ray attenuating wires which intersect at a given interval, the positions of the grid lines in the partial images can be extracted by low-level operations such as disclosed in patent application EP0866342.

If a grid consisting of X-ray attenuating crosses is used, a position x,y in the image with intensity value $I_{x,y}$ could be selected as a possible candidate for a cross if the following conditions are true

$$I_{x+i,y+j} < I_{x+i+d,y+j}$$

$$I_{x+j,y+i} < I_{x+j,y+i+d}$$

$$\forall i : 0 \leq |i| \leq W_1 \quad ,$$

$$\forall j : W_2 \leq |j| \leq L$$

$$\forall d : d_1 \leq |d| \leq d_2$$

where $W_1$ can be interpreted as the central width of the cross lines and $W_2$ as the total width, $L$ as the length of the lines and $d_1, d_2$ as an indication of the size of a region. It is obvious that all these parameters can be tuned to increase the robustness of the detection and that the detection process can be optimized in terms of memory and computation times with standard optimization techniques.

Since such a simple detection mechanism may be prune to generate some false positives, one can accumulate the detected positions by means of a Hough transform to find the period of the grid and to reject the false positives. The positions of the crosses can be further optimized by means of linear regression.

Stitching the images

In the previous section is described how to extract and transform objects of known geometry into a reference plane.

If the same object is present in all the images, the known geometry of the object can be used to stitch the images together accurately.

Suppose an element, A, of the object is detected in a first partial image and an element, B, of the object is detected in a second partial image. Using the determined deformation fields, both positions are mapped onto A' and B' in the transformed partial images. Positions A' and B' are now defined in the reference plane.

If the spatial relationship between A' and B' in the reference plane is known, it is easy to position both partial images in such a way that this spatial relationship is preserved in the combined images. The object of known geometry can thus be used to combine partial images or to combine transformed partial images.

It is furthermore possible to combine transformed partial images on the basis of image information which is not related to the object of known geometry (e.g. visual combination). This may be necessary if the patient has moved between the acquisition of the images.

**Claims**

1. Method of generating a radiation image of an elongate body comprising the steps of

   - generating partial radiation images by multiple shot irradiation and read out of a direct radiography detector, each of said partial radiation images comprising part of the radiation image of said elongate body and part of the radiation image of an object of known geometric dimensions superimposed on the radiation image of said elongate body,
   - calculating parameters of a geometric transformation expressing the relation between detected positions of locations of said object in a partial radiation image and expected positions of said locations in a partial radiation image,
   - projecting said partial images onto a reference plane by applying said geometric transform to its pixels so as to obtain warped partial images,
   - stitching said warped partial images so that the image of said object of known geometric dimensions is reconstructed.

2. A method according to claim 1 wherein said object of known geometric dimensions is a grid consisting of X-ray attenuating wires which intersect at a given interval.

3. A method according to claim 2 wherein said interval is 5 by 5 cm.

4. A method according to claim 1 wherein said object of known geometry is a grid consisting of X-ray attenuating crosses positioned at a given interval.

5. A method according to claim 4 wherein said interval is 5 by 5 cm.

6. A method according to claim 4 wherein said crosses comprise crosses of a first and a second type and wherein all crosses are positioned at a first interval and the crosses of a second type are positioned at a second interval.

7. A method according to claim 6 wherein said first interval is 5 by 5 cm and said second interval is 10 by 10 cm.

8. A method according to claim 1 wherein said geometric transformation is implemented using thin plate spline.

9. A method according to claim 1 wherein said geometric transformation is implemented by piece-wise linear separable de-skewing.

10. A radiographic apparatus for generating a radiation image of an elongate body comprising:

    - an X-ray imaging unit including an X-ray flat panel detector,
    - means for moving said flat panel detector;
    - an X-ray generation unit including an X-ray source,
    - means for moving said X-ray source,
    - an imaging area setting device capable of setting an imaging area for imaging an elongate body;
    - a position determination device for determining a plurality of positions for the X-ray generation unit and the X-ray imaging unit, said positions delineating a plurality of partial imaging areas in said imaging area which overlap with a configured amount,
    - an object of known geometry provided between said X-ray imaging unit and X-ray generation unit, said object comprising parts of X-ray attenuating material distinguishable in images generated by said X-ray imaging unit;
    - at least one control device for controlling said X-ray generation unit and said X-ray imaging unit so that both units are moved sequentially to said positions delineating partial image areas and that partial radiation images of part of said body and part of said object are generated in each of said positions;
    - a processing device for generating an elongate image from the generated partial images.

11. A radiographic apparatus according to claim 10 comprising a diaphragm for collimating the output of said X-ray source.

12. A radiographic apparatus according to claim 10, wherein a patient barrier unit is positioned ahead of the X-ray imaging unit supporting said elongate body, the patient barrier unit being capable of supporting, containing or consisting of the object of known geometry.

13. A radiographic apparatus according to claim 10, wherein said object of known geometry is a grid consisting of X-ray attenuating wires which intersect at a given interval.

14. A radiographic apparatus according to claim 13, wherein said interval is 5 by 5 cm.

15. A radiographic apparatus according to claim 10, wherein said object of know geometry is a grid comprising X-ray attenuating crosses positioned at a given interval.

16. A radiographic apparatus according to claim 15, wherein said interval is 5 by 5 cm.

17. A radiographic apparatus according to claim 15 wherein said crosses comprise crosses of a first and a second type and wherein all crosses are positioned at a first interval and the crosses of a second type are positioned at a second interval.

18. A radiographic apparatus according to claim 18, wherein said first interval is 5 by 5 cm and said second interval is 10 by 10 cm.

19. A radiographic apparatus according to claim 10, wherein said processing device transforms generated partial images to a reference plane before combining them to an elongate image.

20. A radiographic apparatus according to claim 10, wherein said processing device is arranged to generate said elongate image from the generated partial images on the basis of the image of the object of known geometry.

21. A radiographic apparatus according to claim 10, wherein said processing device is arranged to

  - calculate parameters of a geometric transformation expressing the relation between detected positions of locations of said object in a partial radiation image and expected positions of said locations in a partial radiation image, and to
  - project said partial images onto a reference plane by applying said geometric transform to its pixels so as to obtain warped partial images.

22. A radiographic apparatus according to claim 21 wherein said transformation is implemented by using a thin plate spline.

23. A radiographic apparatus according to claim 21 wherein said transformation is implemented by using a piece-wise linear separable de-skewing.

24. A radiographic apparatus according to claim 20, wherein the processing device combines the transformed partial images based on image information not related to the object of known geometry.

25. A radiographic apparatus according to claim 10 wherein said processing device combines said partial images based on image information related to the object of known geometry.

26. A radiographic apparatus according to claim 10 wherein said processing device combines said transformed partial images based on image information related to the object of known geometry.

**FIG. 1**

**FIG. 2**

FIG. 3

**FIG. 4**

FIG. 5

FIG. 6

FIG. 7

**FIG. 8**

**FIG. 9**

Europäisches Patentamt

European Patent Office

Office européen des brevets

## EUROPEAN SEARCH REPORT

Application Number

EP 11 15 7111

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/081010 A1 (CHANG YUN C [US] ET AL) 27 June 2002 (2002-06-27) * the whole document * ----- | 1-26 | INV. A61B6/02 G03B42/02 |
| Y | US 2002/118793 A1 (HORBASCHEK HEINZ [DE]) 29 August 2002 (2002-08-29) * paragraphs [0007] - [0017], [0 25] - [0033] * ----- | 1-26 | |
| Y | US 2002/159564 A1 (WANG XIAOHUI [US] ET AL) 31 October 2002 (2002-10-31) * paragraphs [0002] - [0011] * ----- | 1-26 | |
| Y | EP 2 250 965 A1 (GE MED SYS GLOBAL TECH CO LLC [US]) 17 November 2010 (2010-11-17) * paragraphs [0003], [0007] - [0013], [0020] - [0041] * ----- | 1-26 | |
| Y | EP 0 919 856 A1 (AGFA GEVAERT NV [BE] AGFA GEVAERT [BE]) 2 June 1999 (1999-06-02) * paragraphs [0030] - [0077] * ----- | 1-26 | TECHNICAL FIELDS SEARCHED (IPC) G03B A61B |

The present search report has been drawn up for all claims

| Place of search | Date of completion of the search | Examiner |
|---|---|---|
| Munich | 18 July 2011 | Piedrafita, Aurelio |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 11 15 7111

This annex lists the patent family members relating to the patent documents cited in the above-mentioned European search report.
The members are as contained in the European Patent Office EDP file on
The European Patent Office is in no way liable for these particulars which are merely given for the purpose of information.

18-07-2011

| Patent document cited in search report | | Publication date | Patent family member(s) | | Publication date |
|---|---|---|---|---|---|
| US 2002081010 | A1 | 27-06-2002 | EP 1223751 A1 | | 17-07-2002 |
| | | | US 2005104018 A1 | | 19-05-2005 |
| US 2002118793 | A1 | 29-08-2002 | DE 10109754 A1 | | 26-09-2002 |
| | | | JP 2002306461 A | | 22-10-2002 |
| US 2002159564 | A1 | 31-10-2002 | EP 1263216 A1 | | 04-12-2002 |
| | | | JP 2002336228 A | | 26-11-2002 |
| EP 2250965 | A1 | 17-11-2010 | CN 101884544 A | | 17-11-2010 |
| | | | US 2010290707 A1 | | 18-11-2010 |
| EP 0919856 | A1 | 02-06-1999 | DE 69733689 D1 | | 11-08-2005 |
| | | | DE 69733689 T2 | | 18-05-2006 |
| | | | DE 69825845 D1 | | 30-09-2004 |
| | | | DE 69825845 T2 | | 01-09-2005 |
| | | | JP 11244269 A | | 14-09-1999 |
| | | | JP 4526134 B2 | | 18-08-2010 |
| | | | JP 11244270 A | | 14-09-1999 |
| | | | US 6269177 B1 | | 31-07-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0919856 A
- EP 0866342 A
- DE 102007025448